# FASCICULE DE BREVET EUROPEEN

(11) **EP 2 218 440 B1**
(45) Date de publication et mention de la délivrance du brevet: **08.11.2017**
(21) Numéro de dépôt: 10152370.2
(22) Date de dépôt: 02.02.2010
(51) Int. Cl.: A61K 8/06, A61K 8/37, A61K 8/60, A61Q 1/14, A61Q 19/10

(54) **Composition comprenant un ester de sucrose et un ester de polyglycérol**
Zusammensetzung, die einen Sucroseester und einen Polyglycerolester enthält
Composition including a sucrose ester and a polyglycerol ester

(30) Priorité: 13.02.2009 FR 0950930
(43) Date de publication de la demande: 18.08.2010
(73) Titulaire: L'Oréal, 75008 Paris (FR)
(72) Inventeur: Poletti, Mickaël, 75012, PARIS (FR)
(74) Mandataire: Duvert, Sandra

(56) Documents cités:
- EP-A- 0 289 636
- EP-A- 1 679 063
- EP-A- 1 902 633
- WO-A-2008/140065
- JP-A- 58 103 325
- JP-A- 2005 068 083
- DATABASE WPI Week 19965 Thomson Scientific, London, GB; AN 1996-044286 XP002549513 & JP 07 308562 A (MITSUBISHI CHEM CORP) 28 novembre 1995 (1995-11-28)

## Description

L'invention a pour objet une composition cosmétique comprenant un composé lipophile et un système émulsionnant comprenant un ester de sucrose et d'acide gras et un ester de polyglycérol et d'acide gras, et son utilisation dans le domaine cosmétique, en particulier pour le nettoyage et/ou le démaquillage des matières kératiniques.

Pour obtenir des propriétés d'usage satisfaisantes et/ou une bonne performance produit, il est courant d'introduire dans des composition cosmétiques aqueuses des ingrédients non-miscibles à l'eau comme par exemple de parfum, d'huiles essentielles, d'actifs lipophiles, de filtres solaires ou des corps gras.
Toutefois, du fait de leur caractère lipophile, certains composés sont plus ou moins difficiles à solubiliser dans ces formulations et il peut se produire, durant le stockage, des phénomènes de relargage en surface. De tels phénomènes sont indésirables d'un point de vue stabilité de la formulation et/ou vis-à-vis du confort du consommateur dans la mesure où ils peuvent déstabiliser la composition et/ou affecter l'apparence esthétique du produit (aspect trouble) et/ou entraîner des désagréments cosmétiques à l'application sur la peau et/ou les cheveux ; ils limitent en outre la concentration en actifs dans ces formules ce qui ne permet pas d'obtenir des produits suffisamment efficaces. Afin de solubiliser et stabiliser ces composés lipophiles, il est connu d'utiliser des tensioactifs oxyethylénés issus en partie de la pétrochimie (par exemple l'huile de ricin hydrogénée oxyéthylénée).
Or, les consommatrices sont de plus en plus à la recherche de produits cosmétiques formés, en tout ou partie, de constituants naturels ou d'origine naturelle.
Par « composé naturel », on entend un composé que l'on obtient directement de la terre ou du sol, ou à partir de végétaux ou d'animaux, *via,* le cas échéant, un ou des processus physiques, comme par exemple un broyage, un raffinage, une distillation, une purification ou une filtration.
Par composés « d'origine naturelle », on entend un composé naturel ayant subi un ou des traitements chimiques ou industriels annexes, engendrant des modifications n'affectant pas les qualités essentielles de ce composé et/ou un composé comprenant majoritairement des constituants naturels ayant ou non subi des transformations, comme indiquées ci-dessus.
A titre d'exemple non limitatif de traitement chimique ou industriel annexe engendrant des modifications n'affectant pas les qualités essentielles d'un composé naturel, on peut mentionner ceux autorisés par les organismes de contrôle tels qu'Ecocert (Référentiel des produits cosmétiques biologiques et écologiques, janvier 2003) ou définis dans les manuels reconnus dans le domaine, tels que « Cosmetics and Toiletries Magazine », 2005, vol. 120, 9:10.
Il subsiste donc le besoin de disposer d'un système solubilisant des composés lipophiles compatible avec la formulation de produits cosmétiques « naturels » et/ou « certifiés biologiques », qui permette la formulation de ces composés lipophiles sans limitation de de concentration, de manière à obtenir des formulations stables, efficaces, agréables à l'usage et présentant un aspect esthétique attrayant, notamment un aspect limpide, non trouble.

La demanderesse a découvert qu'un système tensioactif comprenant l'association d'un ester de sucrose et d'acide gras et d'un ester de polyglycérol et d'acides gras permet d'atteindre ces objectifs.
La présente invention a donc pour objet une composition cosmétique comprenant une phase aqueuse, au moins un composé lipophile, et l'association d'au moins un ester de sucrose et d'acide gras et d'au moins un ester de polyglycérol et d'acides gras la composition étant telle que définie dans la revendication 1. La composition selon l'invention présente un aspect limpide ou translucide, tant en étant stable et confortable à l'application.
La composition selon l'invention est destinée à une application topique et contient donc un milieu physiologiquement acceptable. On entend ici par « milieu physiologiquement acceptable » un milieu compatible avec les matières kératiniques telles que la peau, les muqueuses, le cuir chevelu, les yeux et/ou les fibres kératiniques telles que les cils ou les cheveux.

### Composé lipophile

Par "composé lipophile" on entend tout composé organique cosmétique ou dermatologique non miscible à l'eau, susceptible d'être complètement dissous à l'état moléculaire dans une phase grasse liquide ou bien d'être solubilisé sous forme colloïdale (par exemple sous forme micellaire) dans une phase grasse liquide. Ces composés lipophiles sont choisis parmi les anti-bactériens, les antifongiques, les anti-séborrhéiques, les anti-acnéiques, les kératolytiques, les agents cicatrisants, les modificateurs de la pigmentation, les accélérateurs de bronzage, les agents de brunissage artificiel, les liporégulateurs, les agents anti-vieillissement et anti-rides, les émollients, les agents rafraîchissants, les cicatrisants, les protecteurs vasculaires, les répulsifs d'insectes, les déodorants, les agents anti-pelliculaires, les agents anti-chute de cheveux, les huiles essentielles, les parfums, les filtres solaires, les anti-oxydants, les piégeurs de radicaux libres, les agents hydratants ou encore les corps gras liquides à température ambiante (huiles), les corps gras solides à température ambiantes (cires) ou les corps gras semi solides à température ambiante tels que les corps gras pâteux ou les beurres.
On peut notamment citer à titre d'exemples de composés lipophiles :
- les céramides,
- les acides gras essentiels,
- les vitamines telles que la vitamine A (rétinol) ou des esters de celle-ci, la vitamine E ou des esters de celle-ci tels que l'acétate de tocophérol, la vitamine D ou des dérivés de celle-ci et la vitamine F ou de dérivés de celle-ci ;
- les carotènes tels que le β-carotène et les dérivés de ceux-ci tels le lycopène
- les dérivés de l'acide salicylique, notamment ceux décrits dans les documents FR-A-2 581 542, EP-A-378 936 et EP-A-570230,
- les filtres solaires lipophiles comme par exemple les dérivés de triazine, les dérivés de dibenzoylméthane, les benzophénones,
- les huiles essentielles pouvant notamment être choisies parmi les huiles d'eucalyptus, de lavandin, de lavande, de vétiver, de litsea cubeba, de citron, d'orange, de santal, de romarin, de camomille, de sarriette, de noix de muscade, de cannelle, d'hysope, de carvi, d'orange, de mandarine, de genièvre, de girofle, de bergamotte, de géranium, et de cade, de gingembre, ce carotte, de lemongrass ou citronnelle, de rose, de fenouil, de thym, de menthe poivrée,
- et leurs mélanges

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 30 atomes de carbone comme les triglycérides des acides heptanoïque ou octanoïque ou encore, par exemple, les huiles de jojoba, de babassu, de tournesol, d'olive, de noix de coco, de noix du brésil, de marula, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de beurre de karité;

- les esters et les éthers de synthèse, notamment d'acides gras, comme les huiles de formules R¹COOR² et R¹OR² dans laquelle R¹ représente le reste d'un acide gras ou d'un alcool gras comportant de 8 à 29 atomes de carbone, et R² représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam®
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; les polydiméthyl-siloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes ;
- leurs mélanges.

On peut citer en particulier les huiles choisies parmi :
- les esters issus de la réaction d'au moins un acide gras comportant au moins 6 atomes de carbone, de préférence de 6 à 26 atomes de carbone et mieux de 6 à 20 atomes de carbone, encore mieux de 6 à 16 atomes de carbone et d'au moins un alcool comprenant de 1 à 17 atomes de carbone et mieux de 3 à 15 atomes de carbone ; on peut citer notamment le myristate d'isopropyle, le palmitate d'isopropyle le caprate/caprylate d'ethyl2-hexyle (ou caprate/caprylate d'octyle), le palmitate d'éthyl-2-hexyle, le néopentanoate d'isostéaryle, l'isononanoate d'isononyle, le laurate d'hexyle, les esters d'acide lactique et d'alcools gras comprenant 12 ou 13 atomes de carbone, le carbonate de dicaprylyle tel que celui qui est commercialisé sous la dénomination CETIOL CC par la société COGNIS,
- les éthers d'acide gras comprenant de 6 à 20 atomes de carbone tel que le dicaprylyl éther (Cetiol OE de Cognis),
- les éthers de glycérol comprenant de 6 à 12 atomes de carbone comme le 2-éthyl hexyle éther de glycérol (nom INCI : ethylhexylglycerin) tel que le Sensiva SC 50 de la société Schulke & Mayr GmbH,
- les alcanes linéaires volatils, avantageusement d'origine végétale, comprenant de 7 à 17 atomes de carbone, en particulier de 9 à 15 atomes de carbone, et plus particulièrement de 11 à 13 atomes de carbone. A titre d'exemples d'alcanes linéaires volatils convenant à l'invention, on peut mentionner ceux décrits dans la demande de brevet de la société Cognis WO 2007/068371. A titre d'exemple d'alcane linéaire volatil convenant à l'invention, on peut citer le n-nonane (C₉), le n-décane (C₁₀), le n-undécane (C₁₁), le n-dodécane (C₁₂), le n-tridécane (C₁₃), le n-tétradecane (C₁₄), le n-pentadécane (C₁₅), le n-héxadécane (C₁₆) et le n-heptadécane (C₁₇) et leurs mélanges.
Selon un mode de réalisation, on utilisera un mélange d'undécane (C₁₁) et de tridécane (C₁₃) comme obtenu aux exemples 1 et 2 de la demande WO2008/155059 de la Société Cognis.
On peut également citer le n-dodécane (C12) et le n-tétradécane (C14) tels que ceux vendus par Sasol respectivement sous les références PARAFOL 12-97 et PARAFOL 14-97, ainsi que leurs mélanges.

De préférence, l'huile est choisie parmi les huiles d'origine végétales.

Les corps gras semi solides à température ambiante tels que les corps gras pâteux ou les beurres peuvent être des composés hydrocarbonées d'origine végétale tels que des triglycérides liquides d'acides gras comportant de 4 à 30 atomes de carbone. On peut citer par exemple les beurres de karité et de cacao.

En particulier le composé lipophile peut être choisi parmi les parfums, les huiles essentielles, les huiles d'origine végétale, les corps gras pâteux ou les beurres tels que cités ci-dessus et leurs mélanges, en particulier parmi les huiles essentielles et les huiles d'origine végétale.

Le composé lipophile est présent en une teneur allant de 0,001 à 40% en poids par rapport au poids total de la composition, de préférence de 0,01 à 30% en poids et mieux de 0,05 à 20% en poids.
En particulier lorsque le composé lipophile est choisi parmi les huiles essentielles, les huiles d'origine végétale, les corps gras pâteux ou les beurres, il peut être présente dans la composition en une teneur allant de 0,001 à 5% en poids par rapport au poids total de la composition, de préférence de 0,01 à 2% en poids et mieux de 0,05 à 1% en poids.

### Ester de sucrose et d'acide gras

Selon la présente invention, l'ester de sucrose et d'acide gras est choisi parmi les esters issus de la réaction de sucrose(s) (saccharose) et d'acide(s) gras comprenant de 10 à 24 atomes de carbone, de préférence de 12 à 20 atomes de carbone, mieux de 12 à 18 atomes de carbone et encore mieux de 12 à 16 atomes de carbone.
Les acides gras comprenant de 10 à 24 atomes de carbone peuvent être linéaires ou ramifiés, saturés ou insaturés.
Les acides gras peuvent être choisis parmi l'acide oléique, l'acide laurique, l'acide palmitique, l'acide myristique, l'acide stéarique, l'acide linoléique, l'acide caprique, ou leurs mélanges
Selon un mode de réalisation, l'ester de sucrose et d'acide gras est choisi parmi les esters issus de la réaction de sucrose et d'acide gras comprenant de 12 à 18 atomes de carbone, de préférence de 12 à 16 atomes de carbone tels que l'acide laurique et/ou l'acide palmitique comme par exemple le sucrose laurate, le sucrose palmitate ou un mélange.
Les esters de sucrose et d'acides gras peuvent être choisis parmi les mono-, di-, tri- et tétra-esters, les polyesters et leurs mélanges. On utilise de préférence des esters à faible degré d'estérification comme par exemple des monoesters, diesters, triesters de sucrose et d'acide gras ou un mélange. L'ester de sucrose et d'acide gras peut se présenter sous forme d'un mélange d'esters à faible degré d'estérification comme par exemple un mélange de monoester et diester ou un mélanges de monoester, diester et triester.

Dans le cas ou l'on utilise un mélange d'esters de sucrose et d'acide gras, on préfère un mélange dans lequel les esters à faible degré d'estérification, en particulier les monoesters, sont majoritaires et représentent par exemple au moins 50%, de préférence au moins 60% en poids du mélange d'esters de sucrose et d'acide gras.
On peut utiliser en particulier un mélange d'esters de sucrose et d'acides gras comprenant de 12 à 16 atomes de carbone, en particulier une mélange de mono, di et triesters d'acide laurique ou d'acide palmitique, ledit mélange pouvant comprendre de façon minoritaire (en une teneur inférieure ou égale à 40% en poids par rapport au poids du mélange d'esters de sucrose et d'acide gras) des esters de sucrose et d'acides gras dans lequel l'acide gras comprend plus de 16 atomes de carbone.

De préférence, l'ester de sucrose et d'acide gras utilisé dans la présente invention présente une HLB supérieure ou égale à 10, de préférence supérieure ou égale à 12. Comme cela est bien connu, on entend par HLB (Hydrophilic-Lipophilic Balance) l'équilibre entre la dimension et la force du groupe hydrophile et la dimension et la force de groupe lipophile de l'agent tensioactif.
La valeur HLB selon GRIFFIN est définie dans J. Soc. Cosm. Chem. 1954 (volume 5), pages 249-256.

On peut citer à titre d'exemples d'esters ou de mélanges d'esters de sucrose et d'acide gras :
- le Surfhope SE COSME C-1416, présentant une HLB de 16, qui est un sucrose myristate comprenant environ 80% de monoester, le reste du mélange étant composé de di et triesters,
- le Surfhope SE COSME C-1216 dont le nom INCI est sucrose laurate, de HLB égale à 16 et comprend de 75 à 90% de monoester, le reste du mélange étant composé de di et triesters,
- le Surfhope SE COSME C-1215L dont le nom INCI est sucrose laurate, de HLB égale à 15, comprenant environ 70% de monoesters, le reste du mélange étant composé de diesters et autres polyetsers,
- le Surfhope SE COSME C-1616, présentant une HLB de 16, qui est un mélange d'esters de saccharose et d'acides palmitique et/ou stéarique (nom INCI sucrose palmitate), comprenant de 75 à 90% de monoester, le reste du mélange étant composé de di et triesters, et pouvant comprendre du sucrose stéarate et sucrose palmitate stéarate.
On peut également citer l'ester portant le nom INCI sucrose laurate commercialisé par la société Dai-ichi Seiyaku sous la référence DK ester S-L18A, de HLB égale à 17, comprenant 70% de monoesters et 30% de di- et tri-esters.
On peut aussi citer à titre d'exemples d'esters ou de mélanges d'esters de sucrose d'acide gras:
- les produits vendus sous les dénominations F160, F140, F110, F90, F70, SL40 par la société CRODESTA, désignant respectivement les palmito-stéarates de sucrose formés de 73% de monoester et 27% de di- et tri-ester, de 61% de monoester et 39% de di-, tri-, et tétra-ester, de 52% de monoester et 48% de di-, tri-, et tétra-ester, de 45% de monoester et 55% de di-, tri-, et tétra-ester, de 39% de monoester et 61% de di-, tri-, et tétra-ester, et le mono-laurate de sucrose;
- les produits vendus sous la dénomination RYOTO SUGAR ESTERS par exemple référencés B370 et correspondant au béhénate de saccharose formé de 20% de monoester et 80% de di-triester-polyester;
- le mono-di-palmito-stéarate de sucrose commercialisé par la société GOLDSCHMIDT sous la dénomination TEGOSOFT PSE.

La quantité d'ester(s) de sucrose et d'acide gras va de 1 à 5 % en poids par rapport au poids total de la composition.

### Ester de polyglycérol et d'acides gras

Selon la présente invention, l'ester de polyglycérol et d'acide gras est choisi parmi les esters issus de la réaction de polyglycérol comprenant de 2 à 12 unités glycérol, de préférence de 3 à 10 unités glycérol et d'au moins un acide gras comprenant de 8 à 24 atomes de carbone, de préférence de 8 à 20 atomes de carbone, mieux de 10 à 18 atomes de carbone et encore mieux de 10 à 14 atomes de carbone. Les acides gras comprenant de 8 à 24 atomes de carbone peuvent être linéaires ou ramifiés, saturés ou insaturés.

Les acides gras peuvent être choisis parmi l'acide oléique, l'acide stéarique, l'acide isostéarique, l'acide laurique, l'acide palmitique, l'acide myristique, l'acide linoléique, l'acide caprique, l'acide caprylique, ou leurs mélanges.

Les esters de polyglycérol et d'acides gras peuvent être choisis parmi les mono-, di-, tri-et tétra-esters, les polyesters et leurs mélanges. On utilise de préférence des esters à faible degré d'estérification comme par exemple des monoesters, diesters, triesters de sucrose et d'acide gras ou un mélange. L'ester de polyglycérol et d'acide gras peut se présenter sous forme d'un mélange d'esters à faible degré d'estérification comme par exemple un mélange de monoester et diester ou un mélanges de monoester, diester et triester.
Selon un mode de réalisation préféré, on utilise un ester de polyglycérol et d'acide gras choisi parmi les esters issus de la réaction de polyglycérol comprenant de 2 à 12 unités glycérol, de préférence de 4 à 10 unités glycérol, et d'au moins un acide gras comprenant moins de 16 atomes de carbone, de préférence moins de 15 atomes de carbone, par exemple de 8 à 16 atomes de carbone et mieux de 8 à 14 atomes de carbone.

Selon un mode de réalisation, l'ester de polyglycérol et d'acide gras est choisi parmi les esters issus de la réaction de polyglycérol comprenant de 4 à 10 unités glycérol et d'au moins un acide gras comprenant de 8 à 12 atomes de carbone de préférence de 10 à 12 atomes de carbone tels que l'acide laurique et/ou l'acide caprique. On peut citer par exemple l'ester issu de la réaction de polyglycérol-10 (homopolymère de glycérol comprenant 10 unités glycérol) et d'acide laurique (nom INCI : polyglyceryl-10 laurate) tel que celui commercialisé par la société Dr Straetmans sous la référence DERMOFEEL G 10 L, l'ester issu de la réaction de polyglycérol-4 (homopolymère de glycérol comprenant 4 unités glycérol) et d'acide caprique (nom INCI : polyglyceryl-4 caprate) tel que celui commercialisé par la société Evonik sous la référence TEGOSOFT PC 41 .

La quantité d'ester(s) de polyglycérol et d'acide gras va de 1 à 5 % en poids par rapport au poids total de la composition.

Le ratio ester de sucrose et d'acide gras sur ester de polyglycérol et d'acides gras va de 0,5 à 2 et encore mieux de 0,9 à 2, et il est en particulier de l'ordre de 1.

L'association ester de sucrose et d'acide gras et ester de polyglycérol et d'acide gras constitue le système tensioactif non ionique principal de la composition.
Par « système tensioactif non ionique principal », on entend un système qui, en son absence, ne conduit pas à la formation d'une composition stable, c'est-à-dire que les autres tensioactifs nonioniques (différents de l'ester de sucrose et d'acide gras et de l'ester de polyglycérol et d'acide gras) éventuellement présents dans la composition ne conduisent pas à une composition stable en l'absence desdits esters.
Selon un mode de réalisation, l'association ester de sucrose et d'acide gras et ester de polyglycérol et d'acide gras constitue le système tensioactif principal de la composition. Par « système tensioactif principal », on entend un système qui, en son absence, ne conduit pas à la formation d'une composition stable.

Par « stable », on entend une composition qui, soit juste après avoir été formulé, soit après avoir été placée d'une part dans une étuve à 45°C et d'autre part dans un réfrigérateur à 4°C pendant deux mois, ne présente pas, après retour à température ambiante, de déphasage des phases grasse et aqueuse, ou de relargage de phase grasse en surface ou de trouble (si la composition est limpide).
Selon un mode de réalisation particulier, la composition comprend moins de 1% de tensioactif non ionique additionnel (borne incluse) et mieux moins de 0,5% de tensioactif non ionique additionnel (borne incluse). Par tensioactif non ionique additionnel, on entend un tensioactif non ionique différent de l'ester de sucrose et d'acide gras et de l'ester de polyglycérol et d'acide gras décrits plus haut. Selon un mode de réalisation, la composition est exempte de tensioactif non ionique additionnel La composition comprend moins de 1% de tensioactif additionnel (borne incluse) et mieux moins de 0,5% de tensioactif additionnel (borne incluse). Par tensioactif additionnel, on entend tout type de tensioactif, différent de l'ester de sucrose et d'acide gras et de l'ester de polyglycérol et d'acide gras décrits plus haut, y compris les tensioactifs moussants.

Selon un mode de réalisation, l'association ester de sucrose et d'acide gras et ester de polyglycérol et d'acide gras constitue l'unique système tensioactif de la composition, c'est-à-dire que la composition est exempte de tensioactif additionnel.

Selon un mode de réalisation, la composition peut comprendre au moins un agent moussant choisi parmi les tensioactifs anioniques, cationiques, amphotères ou zwitterioniques et leurs mélanges. En ce qui concerne la définition de ce composé, on peut se reporter au document « Encyclopédia of chemical technology, Kirkt-Othmer », volume 22, pages 333-432, 3ème édition 1979, Edition Wiley, où sont citées les principales classes d'agents moussants connues de l'homme du métier, ainsi que leur fonction, en particulier le fait d'être moussant. Ces agents moussants sont distincts des tensioactifs du système tensioactif principal dans la mesure où ils apportent une fonction moussante à la composition. Ils n'ont de préférence pas d'influence à eux seuls sur la stabilisation de la composition.

Les agent moussants peuvent être présents dans la composition selon l'invention en une teneur allant de 0,1 à 30% en poids par rapport au poids total de la composition, de préférence de 0,1 à 20% en poids et mieux de 0,5 à 15% en poids.

Les tensioactifs anioniques peuvent être choisis notamment parmi les dérivés anioniques de protéines d'origine végétale ou de protéines de soie, les phosphates et alkylphosphates, les carboxylates, les sulfosuccinates, les dérivés des aminoacides, les alkyl sulfates, les alkyl éther sulfates, les sulfonates, les iséthionates, les taurates, les alkyl sulfoacétates, les polypeptides, les dérivés anioniques d'alkyl polyglucoside, et leurs mélanges.
1) Les dérivés anioniques de protéines d'origine végétale sont des hydrolysats de protéine à groupement hydrophobe, ledit groupement hydrophobe pouvant être naturellement présent dans la protéine ou être ajouté par réaction de la protéine et/ou de l'hydrolysat de protéine avec un composé hydrophobe. Les protéines sont d'origine végétale ou dérivées de soie, et le groupement hydrophobe peut être notamment une chaîne grasse, par exemple une chaîne alkyle comportant de 10 à 22 atomes de carbone. Comme dérivés anioniques de protéines d'origine végétale, on peut plus particulièrement citer les hydrolysats de protéines de pomme, de blé, de soja, d'avoine, comportant une chaîne alkyle ayant de 10 à 22 atomes de carbone et leurs sels. La chaîne alkyle peut être notamment une chaîne lauryle et le sel peut être un sel de sodium, de potassium et/ou d'ammonium.
   Ainsi, comme hydrolysats de protéine à groupement hydrophobe, on peut citer par exemple les sels des hydrolysats de protéine de soie modifiée par l'acide laurique, tels que le produit commercialisé sous la dénomination KAWA SILK par la société Kawaken ; les sels des hydrolysats de protéine de blé modifiée par l'acide laurique, tels que le sel de potassium commercialisé sous la dénomination Aminofoam W OR par la société Croda (nom INCI : Potassium lauroyl wheat aminoacids) et le sel de sodium commercialisé sous la dénomination PROTEOL LW 30 par la société Seppic (nom INCI : sodium lauroyl wheat aminoacids) ; les sels des hydrolysats de protéine d'avoine comportant une chaîne alkyle ayant de 10 à 22 atomes de carbone, et plus spécialement les sels des hydrolysats de protéine d'avoine modifiée par l'acide laurique, tels que le sel de sodium commercialisé sous la dénomination PROTEOL OAT (solution aqueuse à 30 %) par la société Seppic (nom INCI : Sodium lauroyl oat aminoacids) ; les sels des hydrolysats de protéine de pomme, comportant une chaîne alkyle ayant de 10 à 22 atomes de carbone, tels que le sel de sodium commercialisé sous la dénomination PROTEOL APL (solution hydroglycolique à 30%) par la société Seppic (nom INCI : Sodium Cocoyl Apple amino acids). On peut citer aussi le mélange de lauroyl-aminoacides (aspartique, glutamique, glycine, alanine) neutralisé au N-methylglycinate de sodium, commercialisé sous la dénomination PROTEOL SAV 50 S par la société Seppic (nom INCI : Sodium Cocoyl amino acids).
2) Comme phosphates et alkylphosphates, on peut citer par exemple les monoalkylphosphates et les dialkyl phosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea, le sel de potassium ou de triéthanolamine de mono-alkyl (C₁₂-C₁₃) phosphate commercialisé sous les références ARLATONE MAP 230K-40® et ARLATONE MAP 230T-60® par la société Uniqema, le lauryl phosphate de potassium commercialisé sous la dénomination DERMALCARE MAP XC-99/09® par la société Rhodia Chimie, et le cétylphosphate de potassium commercialisé sous la dénomination ARLATONE MAP 160K par la société Uniqema.
3) Comme carboxylates, on peut citer :
   - Les amido éthercarboxylates (AEC), comme le Lauryl amido ether carboxylate de sodium (3 OE), commercialisé sous la dénomination AKYPO FOAM 30® par la société Kao Chemicals.
   - Les sels d'acides carboxyliques polyoxyéthylénés, comme le Lauryl ether carboxylate de sodium (C₁₂₋₁₄₋₁₆ 65/25/10) oxyéthyléné (6 OE) commercialisé sous la dénomination AKYPO SOFT 45 NV® par la société Kao Chemicals, les acides gras d'origine huile d'olive polyoxyéthylénés et carboxyméthylés commercialisés sous la dénomination OLIVEM 400® par la société BIOLOGIA E TECNOLOGIA, le tri-decyl ether carboxylate de sodium oxyéthyléné (6 OE) commercialisé sous la dénomination NIKKOL ECTD-6NEX® par la société Nikkol.
   - Les sels d'acides gras ayant une chaîne alkyl en C₆ à C₂₂, neutralisés par une base organique ou minérale, qui constituent les savons. Le sel d'acide gras ou savon est obtenu à partir d'un acide gras et d'une base, l'acide gras comportant une chaîne alkyle linéaire ou ramifiée, saturée ou insaturée, ayant de 12 à 22 atomes de carbone et de préférence 12 à 20 atomes de carbone. Les bases (aussi appelées aussi agents de saponification) neutralisent totalement ou partiellement les acides gras. Les bases susceptibles d'être utilisées pour obtenir les sels peuvent être par exemple les bases minérales comme les hydroxydes de métaux alcalins (hydroxyde de sodium et potasse), les hydroxydes de métaux alcalino-terreux (de magnésium) ou l'hydroxyde d'ammonium, ou encore les bases organiques comme la triéthanolamine, la N-méthylglucamine, la lysine et l'arginine. L'acide gras peut être choisi en particulier parmi les acides gras en C₁₀ à C₂₄, et notamment en C₁₂-C₁₈, et en particulier l'acide laurique, l'acide myristique, l'acide stéarique, l'acide palmitique et leurs mélanges.

   Le savon est généralement introduit dans une seconde composition sous forme de la base d'une part et de l'acide gras d'autre part, la formation du sel se faisant in situ.
4) Comme dérivés des aminoacides, on peut citer notamment les sels alcalins d'aminoacides, tels que :
   - les sarcosinates, comme le Lauroyl sarcosinate de sodium commercialisé sous la dénomination SARKOSYL NL 97® par la société Ciba ou commercialisé sous la dénomination ORAMIX L 30® par la société Seppic, le myristoyl sarcosinate de sodium commercialisé sous la dénomination NIKKOL SARCOSINATE MN® par la société Nikkol, le palmitoyl sarcosinate de sodium commercialisé sous la dénomination NIKKOL SARCOSINATE PN® par la société Nikkol.
   - les alaninates, comme le N-lauroyl-N methyl amidopropionate de sodium commercialisé sous la dénomination SODIUM NIKKOL ALANINATE LN 30® par la société Nikkol, ou commercialisé sous la dénomination ALANONE ALE® par la société Kawaken, le N-lauroyl N-methyl alanine triéthanolamine commercialisé sous la dénomination ALANONE ALTA® par la société Kawaken.
   - les glutamates, comme le mono-cocoyl glutamate de triéthanolamine commercialisé sous la dénomination ACYLGLUTAMATE CT-12® par la société Ajinomoto, le lauroylglutamate de triéthanolamine commercialisé sous la dénomination ACYLGLUTAMATE LT-12® par la société Ajinomoto.
   - les aspartates, comme le mélange de N-lauroyl aspartate de triethanolamine / N-myristoyl aspartate de triethanolamine commercialisé sous la dénomination ASPARACK® par la société Mitsubishi.
   - les dérivés de glycine (glycinates), comme le N-cocoyl glycinate de sodium commercialisé sous les dénominations AMILITE GCS-12® et AMILITE GCK 12 par la société Ajinomoto.
   - les citrates tels que le mono-ester citrique d'alcools de coco oxyéthylénés (9 moles), commercialisé sous la dénomination WITCONOL EC 1129 par la société Goldschmidt.
   - les galacturonates tels que le dodeécyl d-galactoside uronate de sodium commercialisé par la société Soliance.
5) Comme sulfosuccinates, on peut citer par exemple le mono-sulfosuccinate d'alcool laurylique (C₁₂/C₁₄ 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C₁₂-C₁₄, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Cognis, le mono-sulfosuccinate d'amide laurique oxyéthyléné (5 OE) commercialisé sous la dénomination
   LEBON A-5000® par la société Sanyo, le sel di-sodique de mono-sulfosuccinate de lauryl citrate oxyéthyléné (10 OE) commercialisé sous la dénomination REWOPOL SB CS 50® par la société Witco, le mono-sulfosuccinate de mono-éthanolamide ricinoléique commercialisé sous la dénomination REWODERM S 1333® par la société Witco. On peut utiliser aussi les sulfosuccinates de polydimethylsiloxane tels que le disodium PEG-12 dimethicone sulfosuccinate commercialisé sous la dénomination MACKANATE-DC30 par la société Mac Intyre.
6) Comme alkyl sulfates, on peut citer par exemple le lauryl sulfate de triéthanolamine (nom INCI : TEA-lauryl sulfate) tel que le produit commercialisé par la société Huntsman sous la dénomination EMPICOL TL40 FL ou celui commercialisé par la société Cognis sur la dénomination TEXAPON T42, produits qui sont à 40 % en solution aqueuse. On peut aussi citer le lauryl sulfate d'ammonium (nom CFTA : Ammonium lauryl sulfate) tel que le produit commercialisé par la société Huntsman sous la dénomination EMPICOL AL 30FL qui est à 30 % en solution aqueuse.
7) Comme alkyl éther sulfates, on peut citer par exemple le lauryl éther sulfate de sodium (nom INCI : sodium laureth sulfate) comme celui commercialisé sous les dénominations TEXAPON N40 et TEXAPON AOS 225 UP par la société Cognis ou comme celui commercialisé sous la dénomination EMPICOL ESB 3/FL3 par la société Huntsman, le lauryl éther sulfate d'ammonium (nom INCI : ammonium laureth sulfate) comme celui commercialisé sous la dénomination STANDAPOL EA-2 par la société Cognis.
8) Comme sulfonates, on peut citer par exemple les alpha-oléfines sulfonates comme l'alpha-oléfine sulfonate de sodium (C₁₄-₁₆) commercialisé sous la dénomination BIO-TERGE AS-40® par la société Stepan, commercialisé sous les dénominations WITCONATE AOS PROTEGE® et SULFRAMINE AOS PH 12® par la société Witco ou commercialisé sous la dénomination BIO-TERGE AS-40 CG® par la société Stepan, l'oléfine sulfonate de sodium secondaire commercialisé sous la dénomination HOSTAPUR SAS 30® par la société Clariant ; les alkyl aryl sulfonates linéaires comme le xylène sulfonate de sodium commercialisé sous les dénominations MANROSOL SXS30®, MANROSOL SXS40®, MANROSOL SXS93® par la société Manro.
9) Comme iséthionates, on peut citer les acyliséthionates comme le cocoyl-iséthionate de sodium, tel que le produit commercialisé sous la dénomination JORDAPON Cl P® par la société Jordan.
10) Comme taurates, on peut citer le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.
11) Les dérivés anioniques d'alkyl-polyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia, le sel di-sodique d'ester sulfosuccinique de cocoylpolyglucoside (1,4), commercialisé sous la dénomination ESSAI 512 MP® par la société Seppic, le sel de sodium d'ester citrique de cocoyl polyglucoside (1,4) commercialisé sous la dénomination EUCAROL AGE-EC® par la société Cesalpinia.

Les tensioactifs amphotères et zwitterioniques peuvent être choisis par exemple parmi les bétaïnes, les N-alkylamidobétaïnes et leurs dérivés, les sultaïnes, les alkyl polyaminocarboxylates, les alkylamphoacétates et leurs mélanges.
1) Comme bétaïnes, on peut citer notamment les alkylbétaïnes comme par exemple la cocobétaïne comme le produit commercialisé sous la dénomination DEHYTON AB-30® par la société Cognis, la laurylbétaïne comme le produit commercialisé sous la dénomination GENAGEN KB® par la société Clariant, la laurylbétaïne oxyethylénée (10 OE), comme le produit commercialisé sous la dénomination LAURYLETHER (10 OE) BETAINE® par la société Shin Nihon Rica, la stéarylbétaïne oxyéthylénée (10 OE) comme le produit commercialisé sous la dénomination STEARYLETHER (10 OE) BETAINE® par la société Shin Nihon Rica.
   Parmi les N-alkylamidobétaines et leurs dérivés, on peut citer par exemple la cocamidopropyl bétaine commercialisée sous la dénomination LEBON 2000 HG® par la société Sanyo, ou commercialisée sous la dénomination EMPIGEN BB® par la société Albright & Wilson, la lauramidopropyl bétaïne commercialisée sous la dénomination REWOTERIC AMB12P® par la société Witco.
2) Comme sultaines, on peut citer les hydroxylsultaïnes, telles que la cocamidopropyl hydroxysultaïne comme le produit commercialisé sous la dénomination REWOTERIC AM CAS par la société Golschmidt-Degussa, ou le produit commercialisé sous la dénomination CROSULTAINE C-50® par la société Croda.
3) Comme alkyl polyaminocarboxylates (APAC), on peut citer le cocoylpolyamino-carboxylate de sodium, commercialisé sous la dénomination AMPHOLAK 7 CX/C® et AMPHOLAK 7 CX® par la société Akzo Nobel, le stéaryl-polyamidocarboxylate de sodium commercialisé sous la dénomination AMPHOLAK 7 TX/C par la société Akzo Nobel, la carboxyméthyloléyl-polypropylamine de sodium, commercialisé sous la dénomination AMPHOLAK XO7/C® par la société Akzo Nobel.
4) Comme alkylamphoacétates, on peut citer par exemple le N-cocoyl-N-carboxyméthoxyéthyl-N-carboxyméthyl-éthylènediamine N-di-sodique (nom INCI: disodium cocoamphodiacetate) comme le produit commercialisé sous la dénomination MIRANOL C2M CONCENTRE NP® par la société Rhodia, le N-cocoyl-N-hydroxyéthyl-N-carboxyméthyl-éthylènediamine N-sodique (nom INCI: sodium cocamphoacetate), le cocoamphohydroxypropyl sulfonate de sodium commercialisé sous la dénomination MIRANOL CSE par la société Rhodia.

Les tensioactifs cationiques utilisables selon la présente invention sont notamment les sels d'amines grasses primaires, secondaires ou tertiaires, éventuellement polyoxyalkylénées ; les sels d'ammonium quaternaire ; les dérivés d'imidazoline ; les oxydes d'amines à caractère cationique, et/ou l'un de leurs mélanges.

Les sels d'ammonium quaternaires sont par exemple :
- ceux qui présentent la formule générale (IV) suivante : 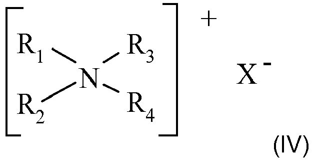 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates, De préférence R₁ et R₂ désigne un alkyle en C₁-C₄, ou un hydroxyalkyle en C₁-C₄.
- les sels d'ammonium quaternaire de l'imidazolinium, comme par exemple celui de formule (V) suivante : 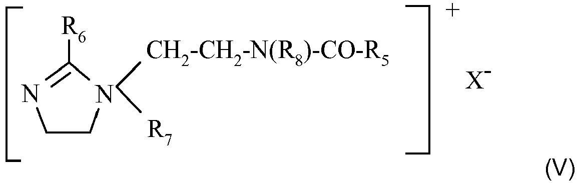 dans laquelle R₅ représente un radical alcényle ou alkyle comportant de 8 à 30 atomes de carbone par exemple dérivés des acides gras de coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X⁻ est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène.
- les sels de diammonium quaternaire de formule (VI) : 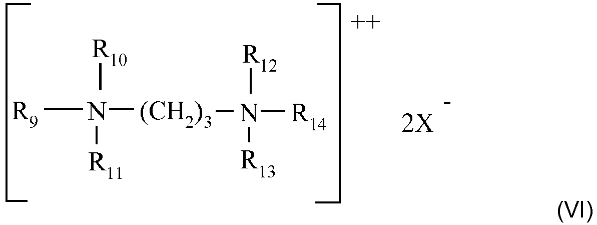 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates.
- les sels d'ammonium quaternaire contenant au moins une fonction ester par exemple ceux de formule (VII) suivante : 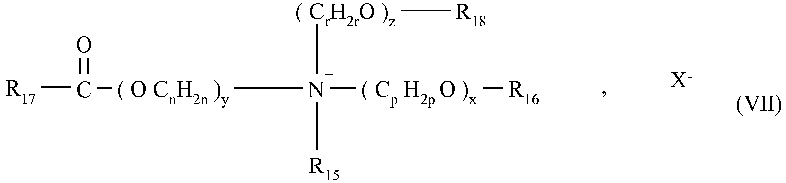 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
      - le radical 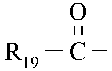
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 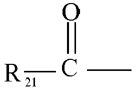
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X- est un anion simple ou complexe, organique ou inorganique ;
Sous réserve que la somme x + y + z vaut de 1 à 15, que lorsque x vaut 0 alors R₁₆ désigne R₂₀ et que lorsque z vaut 0 alors R₁₈ désigne R₂₂.
Les radicaux alkyles R₁₅ peuvent être linéaires ou ramifiés et plus particulièrement linéaires.
De préférence R₁₅ désigne un radical méthyle, éthyle, hydroxyéthyle ou dihydroxypropyle et plus particulièrement un radical méthyle ou éthyle. Avantageusement, la somme x + y + z vaut de 1 à 10.
Lorsque R₁₆ est un radical R₂₀ hydrocarboné, il peut être long et avoir de 12 à 22 atomes de carbone ou court et avoir de 1 à 3 atomes de carbone.
Lorsque R₁₈ est un radical R₂₂ hydrocarboné, il a de préférence 1 à 3 atomes de carbone.
Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.
L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.
L'anion X- est encore plus particulièrement le chlorure ou le méthylsulfate.
On utilise plus particulièrement les sels d'ammonium de formule (VII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 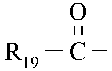
   - les radicaux méthyle, éthyle ou hydrocarbonés en C₁₄-C₂₂
   - l'atome d'hydrogène ;
- R₁₈ est choisi parmi :
   - le radical 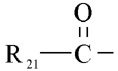
   - l'atome d'hydrogène ;

R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés. Avantageusement, les radicaux hydrocarbonés sont linéaires.

Parmi les sels d'ammonium quaternaire de formule (IV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyldiméthylstéaryl ammonium ou encore, d'autre part, le chlorure de palmitylamidopropyltriméthylammonium ou le chlorure de stéaramidopropyldiméthyl (myristyl acétate) ammonium commercialisé sous la dénomination "CERAPHYL 70" par la société VAN DYK.
On peut citer par exemple les composés de formule (V) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.
Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.
De tels composés sont par exemple commercialisés sous les dénominations DEHYQUART par la société COGNIS, STEPANQUAT par la société STEPAN, NOXAMIUM par la société CECA, REWOQUAT WE 18 et REWOQUAT W75 par la société DEGUSSA.
On peut aussi utiliser les sels d'ammonium contenant au moins une fonction ester décrits dans les brevets US-A-4874554 et US-A-4137180.
Des sels de diammonium quaternaire de formule (VI) convenant à l'invention comprennent notamment le dichlorure de propanesuif diammonium.

### Phase aqueuse

La composition selon l'invention comprend une phase aqueuse comprenant de l'eau et/ou des solvants hydrophiles comme des polyols.
L'eau est présente en une quantité allant de 30 à 95 % en poids en poids, et encore mieux allant de 30 à 90 % en poids.
L'eau utilisée dans la composition de l'invention peut être de l'eau pure déminéralisée mais aussi de l'eau minérale et/ou de l'eau thermale et/ou de l'eau de mer, c'est-à-dire que l'eau de la composition peut être en partie ou totalement constituée par une eau choisie parmi les eaux minérales, les eaux thermales, les eaux de mer et leurs mélanges. En général, une eau minérale est propre à la consommation, ce qui n'est pas toujours le cas d'une eau thermale. Chacune de ces eaux contient, entre autre, des minéraux solubilisés et/ou des oligo-éléments. Ces eaux sont connues pour être employées à des fins de traitement spécifique selon les oligo-éléments et les minéraux particuliers qu'elles contiennent, tel que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou contenant des oligo-éléments préparées à partir d'eau purifiée (déminéralisée ou distillée).
Une eau thermale ou minérale naturelle utilisée selon l'invention peut, par exemple, être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.
La phase aqueuse de la composition de l'invention peut comprendre un ou plusieurs polyols. Parmi les polyols utilisables dans la composition selon l'invention, on peut citer notamment la glycérine, le propylène glycol, le butylène glycol, l'hexylène glycol, les polyéthylène glycols tels que le PEG-8, le dipropylène glycol et leurs mélanges. Selon un mode préféré de réalisation de l'invention, le polyol est la glycérine qui donne une meilleure transparence de la composition que les autres polyols. On peut ajouter à la glycérine, d'autres polyols dans la mesure où les qualités de la composition et notamment la limpidité sont maintenues.

La quantité de polyol(s) peut aller par exemple de 0,5 à 15 % en poids, de préférence de 0,5 à 10 % en poids, mieux de 1 à 10 % en poids, encore mieux de 2 à 10 % en poids et encore mieux de 2 à 8 % en poids par rapport au poids total de la composition.

La composition peut comprendre un gélifiant hydrophile, choisi de préférence parmi les gélifiants d'origine naturelle, en particulier d'origine végétale, ou les polysaccharides d'origine biotechnologique (par exemple la gomme de xanthane).
Ce polysaccharide issu du végétal peut le cas échéant être modifié chimiquement pour favoriser sa valence hydrophile, comme c'est le cas des dérivés de cellulose, en particulier des hydroxyalkyle celluloses (ex : hydroxyethylcellulose).

Comme exemples de polysaccharides d'origine végétale utilisables selon l'invention, on peut citer notamment :
a) des extraits d'algues, tels que les alginates, les carraghénanes, les agars agars, et leurs mélanges. A titre d'exemples de carraghénanes, on peut citer les Satiagum UTC30® et UTC10® de la société Degussa ; comme alginates, on peut citer l'alginate de sodium vendu sous la dénomination Kelcosol® par la société ISP ;
b) des gommes, telles que la gomme de guar et leurs dérivés non ioniques (hydroxypropyl guar), la gomme arabique, la gomme de konjac ou mannane, la gomme Tragacanthe, la gomme Ghatti, la gomme Karaya, la gomme de caroube ; comme exemples, on peut citer la gomme de guar commercialisée sous la dénomination Jaguar HP105® par la société Rhodia ; la gomme de mannane et konjac® (1% de glucomannane) commercialisée par la société GfN ;
c) les amidons modifiés ou non, tels que ceux issus, par exemple, de céréales comme le blé, le maïs ou le riz, de légumes comme le pois blond, de tubercules comme les pommes de terre ou le manioc, les amidons de tapioca ; des dextrines, telles que les dextrines de mais ; comme exemples, on peut citer notamment l'amidon de riz Remy DR I® commercialisé par la société Remy ; l'amidon de maïs B® de la société Roquette ; la fécule de pomme de terre modifiée par l'acide 2-chloroethyl aminodipropionique neutralisé à la soude commercialisé sous la dénomination Structure Solanace® par la société National Starch ; la poudre d'amidon de tapioca natif commercialisée sous la dénomination Tapioca pure® par la société National Starch ;
d) les dextrines, telles que la dextrine extraite de maïs sous la dénomination Index® de la société National Starch ;
e) les celluloses et leurs dérivés, en particulier les alkyle ou hydroxyalkyle celluloses ; on peut citer notamment les méthylcelluloses, hydroxyalkylcelluloses, éthylhydroxyéthylcelluloses, carboxyméthyl-celluloses. Comme exemples, on peut citer les cetyl hydroxy ethyl cellulose sous les dénominations Polysurf 67CS® et Natrosol Plus 330® d'Aqualon ;
et leurs mélanges.

Selon un mode de réalisation, la composition selon l'invention comprend moins de 1,5% en poids de polymères épaississants ou gélifiants synthétiques, de préférence moins de 1%, mieux moins de 0,5%, voire moins de 0,2% en poids. Elle peut être totalement exempte de polymères épaississants ou gélifiants synthétiques.
De tels polymères synthétiques sont par exemple des polymères acryliques (famille des Carbopol), des copolymères acryliques/alkyl acrylates ou des (co)polymères à base d'acide 2-acrylamido 2-méthylpropane sulfonique (par exemple les polymères commercialisés sous la dénomination Pemulen, Sepigel ou Simulgel, Aristoflex).

Les compositions cosmétiques de l'invention peuvent, en outre, contenir des adjuvants habituels dans le domaine cosmétique, tels que les antioxydants, les conservateurs, les, les matières colorantes, les charges, les actifs hydrophiles. La nature des adjuvants et leurs quantités doivent être telles qu'elles ne modifient pas les propriétés de la composition selon l'invention. Les quantités de ces adjuvants sont celles classiquement utilisées dans le domaine cosmétique et par exemple de 0,001 à 10 % du poids total de la composition.

Comme actifs utilisables dans la composition de l'invention, on peut citer par exemple les agents apaisants comme l'allantoïne et le bisabolol ; les eaux florales telles que l'eau de tilleul ou l'eau de bleuet ; l'acide glycyrrhétinique et ses sels ; les antibactériens comme l'octopirox, le triclosan et le triclocarban ; les co-enzymes tels que le co-enzyme Q10 ou ubiquinone et le co-enzyme R ou biotine ; les hydrolysats de protéine ; les extraits végétaux et notamment les extraits de plancton ; et leurs mélanges.

Bien entendu, l'homme de l'art veillera à choisir le ou les éventuels additifs à ajouter à la composition selon l'invention de manière telle que les propriétés avantageuses attachées intrinsèquement à la composition conforme à l'invention ne soient pas, ou substantiellement pas, altérées par l'addition envisagée.
Comme charges, on peut citer les charges minérales telles que le talc ou silicate de magnésium (granulométrie: 5 microns) commercialisé sous la dénomination LUZENAC 15 M00® par la société LUZENAC, le kaolin ou silicate d'aluminium comme par exemple celui commercialisé sous la dénomination KAOLIN SUPREME® par la société IMERYS, ou les charges organiques telles que l'amidon comme par exemple le produit commercialisé sous la dénomination AMIDON DE MAIS B ® par la société ROQUETTE, les microsphères de Nylon comme celles commercialisées sous la dénomination ORGASOL 2002 UD NAT COS® par la société ATOCHEM, les microsphères à base de copolymère de chlorure de vinylidene/Acrylonitrile/methacrylonitrile enfermant de l'isobutane, expansées comme celles commercialisées sous la dénomination EXPANCEL 551 DE® par la société EXPANCEL. On peut ajouter aussi à la composition de l'invention des fibres comme par exemple des fibres de nylon (POLYAMIDE 0.9 DTEX 0.3 MM commercialisé par les Etablissements PAUL BONTE, des fibres de cellulose ou « Rayonne » (RAYON FLOCK RCISE NOOO3 MO4® commercialisé par la société CLAREMONT FLOCK CORPORATION).

Selon un mode particulier de réalisation de l'invention, la composition selon l'invention contient comme charges, des particules exfoliantes qui vont permettre le gommage de la peau. Comme particules exfoliantes, on peut utiliser des particules exfoliantes ou gommantes d'origines minérale, végétale ou organique. Ainsi, on peut utiliser par exemple les billes ou la poudre de polyéthylène, comme celles commercialisées sous la dénomination Microthene MN 727 ou Microthene MN 710-20 par la société Equistar ou comme la poudre commercialisée sous la dénomination Gotalene 120 Incolore 2 par la société Dupont ; les particules de Nylon comme celles commercialisées par la société Arkema sous la dénomination Orgasol 2002 EXD NAT COS ; les fibres comme les fibres de polyamide, comme celles commercialisées par la société Utexbel sous la dénomination PULPE POLYAMIDE 12185 TAILLE 0,3 MM ; la poudre de polychlorure de vinyle ; la pierre ponce (nom INCI : pumice) comme le ponce 3/B d'Eyraud ; les coques de noyaux de fruits broyées comme les broyats de noyaux d'abricots ou de coques de noix ; la sciure de bois ; les billes de verre ; l'alumine (oxyde d'aluminium) (nom INCI : Alumina) comme le produit commercialisé sous la dénomination Dermagrain 900 par la société Marketech International, ;les cristaux de sucre ; des billes qui fondent lors de l'application sur la peau, telles que par exemple, les sphères à base de mannitol et cellulose commercialisées sous les dénominations Unisphères par la société Induchem, les capsules à base d'agar commercialisées sous les dénominations Primasponge par la société Cognis, et les sphères à base d'esters de jojoba commercialisées sous les dénominations Florasphères par la société Floratech ; et leurs mélanges.

Les compositions selon l'invention peuvent constituer notamment des produits de nettoyage ou de démaquillage des matières kératiniques telles la peau (corps, visage, yeux, cuir chevelu) et/ou des fibres kétatiniques. Elle peut être un par exemple un tonique, une crème nettoyante moussante pour le visage, un lait démaquillant, un biphase, un shampoing.
La composition peut être une composition essentiellement aqueuse, elle peut aussi se présenter sous forme d'une émulsion, il peut s'agir d'une émulsion eau-dans-huile (E.H) ou huile-dans-eau (H/E), ou d'une émulsion multiple (E/H/E ou H/E/H).

Selon un mode de réalisation particulier, la composition selon l'invention est une composition essentiellement aqueuse, comprenant par exemple au moins 85% en poids d'eau par rapport au poids total de la composition, de préférence au moins 90% en poids et mieux au moins 95% en poids. Selon ce mode de réalisation, l'association ester de sucrose et d'acide gras et ester de polyglycérol et d'acide gras constitue le système tensioactif non ionique principal de la composition, comme défini plus haut. En particulier, l'association ester de sucrose et d'acide gras et ester de polyglycérol et d'acide gras constitue le système tensioactif principal de la composition, voire l'unique système tensioactif de la composition, comme défini plus haut.

Un autre objet de l'invention est un procédé de nettoyage ou de démaquillage des matières kératiniques telles que la peau, y compris du cuir chevelu, des fibres kératiniques telles que les cils, les cheveux, et/ou des lèvres, caractérisé par le fait qu'on applique sur lesdites matières kératiniques, une composition cosmétique telle que définie ci-dessus.
Selon un mode de réalisation, ce procédé comprend, une étape de rinçage à l'eau de matières kératiniques.

Un autre objet de l'invention consiste en l'utilisation cosmétique de la composition telle que définie ci-dessus, comme produits de nettoyage et/ou de démaquillage des matières kératiniques.

Un autre objet de l'invention consiste en un procédé cosmétique de nettoyage des matières kératiniques, caractérisé par le fait qu'on applique la composition de l'invention, sur matières kératiniques, en présence d'eau, et qu'on élimine le dépôt formé et les résidus de salissure par rinçage à l'eau.

Dans le cas du nettoyage du visage, la composition selon l'invention peut constituer un masque qui est rincé après un temps de pose de 1 à 3 minutes.

Les exemples suivants sont donnés à titre d'illustration de l'invention et n'ont pas de caractère limitatif. Toutes les quantités sont données en pourcentage en poids par rapport au poids total de la composition. Les noms des composés sont indiqués selon les cas en noms chimiques ou en noms INCI.

### Exemples 1 à 5 : Toniques

| | **Ex 1** | **Ex 2** | **Ex 3** | **Ex 4** | **Ex 5** |
|---|---|---|---|---|---|
| | ***hors invention*** | ***hors invention*** | ***hors invention*** | ***Invention*** | ***Invention*** |
| ***Phase A*** | | | | | |
| Polyglyceryl-10 laurate (DERMOFEEL G 10 L de DR STRAETMANS) | 2,8 MA | - | 1,4 MA | 1,4 MA | - |
| Polyglyceryl-4 caprate (TEGOSOFT PC 41 d'EVONIK) | - | 2,8 MA | 1,4 MA | - | 1,4 MA |
| Sucrose laurate (SURFHOPE SE COSME C-1216 de MITSUBISHI) | - | - | - | 1,4 MA | 1,4 MA |
| Huile de Babassu (Orbignya Oelifera Seed Oil) (CROPURE BABASSU de Croda) | 0,01 | 0,01 | 0,01 | 0,01 | 0,01 |
| Huile essentielle de Géranium bourbon bio (Pelargonium graveolens Flower Oil) (Sanoflore) | 0,15 | 0,15 | 0,15 | 0,15 | 0,15 |

| ***Phase B*** | | | | | |
|---|---|---|---|---|---|
| Glycerine | 3 | 3 | 3 | 3 | 3 |
| Sodium benzoate | 0,35 | 0,35 | 0,35 | 0,35 | 0,35 |
| Potassium sorbate | 0,15 | 0,15 | 0,15 | 0,15 | 0,15 |
| acide citrique | qsp pH 5,4 | qsp pH 5,4 | qsp pH 5,4 | qsp pH 5,4 | qsp pH 5,4 |
| Eau | qsp 100 | qsp 100 | qsp 100 | qsp 100 | qsp 100 |
| | | | | | |
| **Aspect :** | Trouble | Trouble | Trouble | Quasi-limpide (très légèrement voilé) | Limpide |

| | | | | | |
|---|---|---|---|---|---|
| MA = Matières Actives | | | | | |

### Mode opératoire

Les ingrédients de la phase A sont mélangés et chauffés jusqu'à obtenir un mélange homogène.

Le mélange est ensuite ajouté à la phase B dont les ingrédients ont été pré-mélangés à température ambiante.

Le système tensioactif ester de sucrose et d'acides gras + un ester de polyglycérol et d'acide gras selon l'invention selon l'invention permet bien d'obtenir des compositions d'aspect limpide.

## Revendications

1. Composition cosmétique comprenant :
- une phase aqueuse comprenant de l'eau en une teneur allant de 30 à 95 % en poids par rapport au poids total de la composition,
- au moins un composé lipophile en une teneur allant de 0,001 à 40% en poids par rapport au poids total de la composition et choisi parmi les anti-bactériens, les antifongiques, les anti-séborrhéiques, les anti-acnéiques, les kératolytiques, les agents cicatrisants, les modificateurs de la pigmentation, les accélérateurs de bronzage, les agents de brunissage artificiel, les liporégulateurs, les agents anti-vieillissement et anti-rides, les émollients, les agents rafraîchissants, les cicatrisants, les protecteurs vasculaires, les répulsifs d'insectes, les déodorants, les agents anti-pelliculaires, les agents anti-chute de cheveux, les huiles essentielles, les parfums, les filtres solaires, les anti-oxydants, les piégeurs de radicaux libres, les agents hydratants ou encore les corps gras liquides à température ambiante, les corps gras solides à température ambiantes ou les corps gras semi solides à température ambiante et leurs mélanges, et
- un système tensioactif comprenant l'association
- d'au moins un ester de sucrose et d'acide gras choisi parmi les esters issus de la réaction de sucrose(s) et d'acide(s) gras comprenant de 10 à 24 atomes de carbone présent en une quantité allant de 1 à 5 % en poids par rapport au poids total de la composition et
- d'au moins un ester de polyglycérol et d'acides gras choisi parmi les esters issus de la réaction de polyglycérol comprenant de 2 à 12 unités glycérol, et d'au moins un acide gras comprenant moins de 16 atomes de carbone, présent en une quantité allant de 1 à 5 % en poids par rapport au poids total de la composition,
le ratio ester de sucrose et d'acide gras sur ester de polyglycérol et d'acides gras allant de 0,5 à 2, ladite composition comprenant moins de 1% de tensioactif additionnel.

2. Composition selon la revendication 1, **caractérisée en ce que** l'ester de sucrose et d'acide gras est choisi parmi les esters issus de la réaction de sucrose(s) et d'acide(s) gras comprenant de 12 à 20 atomes de carbone, mieux de 12 à 18 atomes de carbone et encore mieux de 12 à 16 atomes de carbone.

3. Composition selon la revendication 1 ou 2, **caractérisée en ce que** l'acide gras de l'ester de sucrose et d'acide gras est choisi parmi l'acide oléique, l'acide laurique, l'acide palmitique, l'acide myristique, l'acide stéarique, l'acide linoléique, l'acide caprique ou leurs mélanges.

4. Composition selon l'une quelconque des revendications précédentes, **caractérisée en ce que** l'ester de sucrose et d'acide gras est choisi parmi les esters issus de la réaction de sucrose et d'acide gras comprenant de 12 à 18 atomes de carbone, de préférence de 12 à 16 atomes de carbone.

5. Composition selon l'une quelconque des revendications précédentes, **caractérisée en ce que** l'ester de sucrose et d'acide gras est choisi parmi le sucrose laurate, le sucrose palmitate ou leur mélange.

6. Composition selon l'une quelconque des revendications précédentes, **caractérisée en ce que** l'ester de polyglycérol et d'acide gras est choisi parmi les esters issus de la réaction de polyglycérol comprenant de 3 à 10 unités glycérol et d'au moins un acide gras comprenant de 10 à 14 atomes de carbone.

7. Composition selon la revendication 6, **caractérisée en ce que** l'acide gras de l'ester de polyglycérol et d'acide gras est choisi parmi l'acide laurique, l'acide myristique, l'acide caprique, l'acide caprylique, ou leurs mélanges.

8. Composition selon l'une quelconque des revendications précédentes, **caractérisée en ce que** l'ester de polyglycérol et d'acide gras est choisi parmi les esters issus de la réaction de polyglycérol comprenant de 4 à 10 unités glycérol, et d'au moins un acide gras comprenant moins de 15 atomes de carbone.

9. Composition selon l'une quelconque des revendications précédentes, **caractérisée en ce que** l'ester de polyglycérol et d'acide gras est choisi parmi les esters issus de la réaction de polyglycérol comprenant de 4 à 10 unités glycérol et d'au moins un acide gras comprenant de 8 à 12 atomes de carbone de préférence de 10 à 12 atomes de carbone tels que l'acide laurique et/ou l'acide caprique.

10. Composition selon l'une quelconque des revendications précédentes, **caractérisée en ce que** l'ester de polyglycérol et d'acide gras est choisi parmi l'ester issu de la réaction de polyglycérol-10 et d'acide laurique, l'ester issu de la réaction de polyglycérol-4 et d'acide caprique et leurs mélanges.

11. Composition selon l'une quelconque des revendications précédentes, **caractérisée en ce que** le ratio ester de sucrose et d'acide gras sur ester de polyglycérol et d'acides gras va de 0,9 à 2.

12. Composition selon l'une quelconque des revendications précédentes, **caractérisée en ce que** composé lipophile est choisi parmi les parfums, les huiles essentielles, les huiles d'origine végétale, les corps gras pâteux ou les beurres et leurs mélanges.

13. Composition selon l'une quelconque des revendications précédentes, **caractérisée en ce que** composé lipophile est présent en une teneur allant de 0,01 à 30% en poids et mieux de 0,05 à 20% en poids par rapport au poids total de la composition.

14. Composition selon l'une quelconque des revendications précédentes, **caractérisée en ce que** le composé lipophile est choisi parmi les huiles essentielles, les huiles d'origine végétale, les corps gras pâteux ou les beurres et est présent en une teneur allant de 0,001 à 5% en poids, par rapport au poids total de la composition, de préférence de 0,01 à 2% en poids et mieux de 0,05 à 1% en poids.

15. Composition selon l'une quelconque des revendications précédentes, **caractérisée en ce que** le composé lipophile est choisi parmi les huiles essentielles, les huiles d'origine végétale et est présent en une teneur allant de 0,001 à 5% en poids, par rapport au poids total de la composition, de préférence de 0,01 à 2% en poids et mieux de 0,05 à 1% en poids.

16. Procédé de nettoyage ou de démaquillage des matières kératiniques, **caractérisé par le fait qu'**on applique sur lesdites matières kératiniques, une composition cosmétique selon l'une des revendications 1 à 15.

## Patentansprüche

1. Kosmetische Zusammensetzung, umfassend:
- eine wässrige Phase, die Wasser umfasst, in einem Gehalt im Bereich von 30 bis 95 Gew.-%, bezogen auf das Gesamtgewicht der Zusammensetzung,
- mindestens eine lipophile Verbindung in einem Gehalt im Bereich von 0,001 bis 40 Gew.-%, bezogen auf das Gesamtgewicht der Zusammensetzung, die ausgewählt ist aus antibakteriellen Mitteln, Antimykotika, Antiseborrhoika, Antiaknemitteln, keratolytika, die Narbenbildung fördernden Mitteln, Pigmentierungsmodifikatoren, Bräunungsbeschleunigern, Mitteln zur künstlichen Bräunung, Liporegulatoren, Mitteln gegen Alterung und gegen Falten, Emollientien, Erfrischungsmitteln, die Narbenbildung fördernden Mitteln, Gefäßschutzmitteln, Insektenschutzmitteln, Desodorantien, Mitteln gegen Schuppen, Mitteln gegen Haarausfall, ätherischen Ölen, Duftstoffen, Lichtschutzmitteln, Antioxidantien, Radikalfängern, Feuchtigkeitsspendern oder auch bei Umgebungstemperatur flüssigen Fettsubstanzen, bei Umgebungstemperatur festen Fettsubstanzen oder bei Umgebungstemperatur halbfesten Festsubstanzen und Mischungen davon, und
- ein Tensidsystem, umfassend die Kombination
- von mindestens einem Saccharosefettsäureester, ausgewählt aus den Estern, die sich aus der Reaktion von Saccharose(n) und Fettsäure(n) mit 10 bis 24 Kohlenstoffatomen ergeben, der in einer Menge im Bereich von 1 bis 5 Gew.-%, bezogen auf das Gesamtgewicht der Zusammensetzung, vorlegt, und
- mindestens einem Polyglycerinfettsäureester, ausgewählt aus den Estern, die sich aus der Reaktion von Polyglycerin mit 2 bis 12 Glycerin-Einheiten und mindestens einer Fettsäure mit mindestens 16 Kohlenstoffatomen ergeben, der in einer Menge im Bereich von 1 bis 5 Ges.-%, bezogen auf das Gesamtgewicht der Zusammensetzung, vorliegt,
wobei das Verhältnis von Saccharosefettsäureester zu Polyglycerinfettsäureester im Bereich von 0,5 bis 2 liegt, wobei die Zusammensetzung weniger als 1% zusätzliches Tensid umfasst.

2. Zusammensetzung nach Anspruch 1, **dadurch gekennzeichnet, dass** der Saccharosefettsäureester aus den Estern, die sich aus der Reaktion von Saccharose(n) und Fettsäure(n) mit 12 bis 20 Kohlenstoffatomen, besser 12 bis 18 Kohlenstoffatomen und noch besser 12 bis 16 Kohlenstoffatomen ergeben, ausgewählt ist.

3. Zusammensetzung nach Anspruch 1 oder 2, **dadurch gekennzeichnet, dass** die Fettsäure des Saccharosefettsäureesters aus Ölsäure, Laurinsäure, Palmitinsäure, Myristinsäure, Stearinsäure, Linolsäure, Capronsäure und Mischungen davon ausgewählt ist.

4. Zusammensetzung nach einem der vorhergehenden Anspruche, **dadurch gekennzeichnet, dass** der Saccharosefettsäureester aus den Estern, die sich aus der Reaktion von Saccharose und einer Fettsäure mit 12 bis 18 Kohlenstoffatomen und vorzugsweise 12 bis 16 Kohlenstoffatomen ergeben, ausgewählt ist.

5. Zusammensetzung nach einem der vorhergehenden Ansprüche, **dadurch gekennzeichnet, dass** der Saccharosefettsäureester aus Saccharoselaurat, Saccharosepalmitat oder einer Mischung davon ausgewählt ist.

6. Zusammensetzung nach einem der vorhergehenden Ansprüche, **dadurch gekennzeichnet, dass** der Polyglycerinfettsäureester aus den Estern, die sich aus der Reaktion von Polyglycerin mit 3 bis 10 Glycerin-Einheiten und mindestens einer Fettsäure mit 10 bis 14 Kohlenstoffatomen ergeben, ausgewählt ist.

7. Zusammensetzung nach Anspruch 6, **dadurch gekennzeichnet, dass** die Fettsäure des Polyglycerinfettsäureesters aus Laurinsäure, Myristinsäure, Caprinsäure, Caprylsäure oder Mischungen davon ausgewählt ist.

8. Zusammensetzung, nach einem der vorhergehenden Ansprüche, **dadurch gekennzeichnet, dass** der Polyglycerinfettsäureester aus den Estern, die sich aus der Reaktion von Polyglycerin mit 4 bis 10 Glycerin-Einheiten und mindestens einer Fettsäure mit weniger als 15 Kohlenstoffatomen ergeben, ausgewählt ist.

9. Zusammensetzung nach einem der vorhergehenden Ansprüche, **dadurch gekennzeichnet, dass** der Polyglycerinfettsäureester aus den Estern, die sich aus der Reaktion von Polyglycerin mit 4 bis 10 Glycerin-Einheiten und mindestens einer Fettsäure mit 8 bis 12 Kohlenstoffatomen und vorzugsweise 10 bis 12 Kohlenstoffatomen wie Laurinsäure und/oder Caprinsäure ergeben, ausgewählt ist.

10. Zusammensetzung nach einem der vorhergehenden Ansprüche, **dadurch gekennzeichnet, dass** der Polyglycerinfettsäureester aus dem Ester, der sich aus der Reaktion von Polyglycerin-10 und Laurinsäure ergibt, dem Ester, der sich aus der Reaktion von Polyglycerin-4 und Caprinsäure ergibt, und Mischungen davon ausgewählt ist.

11. Zusammensetzung nach einem der vorhergehenden Ansprüche, **dadurch gekennzeichnet, dass** das Verhältnis von Saccharosefettsäureester zu Polyglycerinfettsäureester im Bereich von 0,9 bis 2 liegt.

12. Zusammensetzung, nach einem der vorhergehenden Ansprüche, **dadurch gekennzeichnet, dass** die lipophile Verbindung aus Duftstoffen, ätherischen Ölen, Ölen pflanzlicher Herkunft, pastösen Fettsubstanzen oder Buttern und Mischungen davon ausgewählt ist.

13. Zusammensetzung nach einem der vorhergehenden Ansprüche, **dadurch gekennzeichnet, dass** die lipophile Verbindung in einem Gehalt im Bereich von 0,01 bis 30 Ges.-% und besser 0,05 bis 20 Gew.-%, bezogen auf das Gesamtgewicht der Zusammensetzung, vorliegt.

14. Zusammensetzung nach einem der vorhergehenden Ansprüche, dadurch gekenntzeichnet, dass die lipophile Verbindung aus ätherischen Ölen, Ölen pflanzlicher Herkunft, pastösen Fettsubstähzen öder Buttern ausgewählt ist und in einem Gehalt im Bereich von 0,001 bis 5 Gew.-%, bezogen auf das Gesamtgewicht der Zusammensetzung, vorzugsweise 0,01 bis 2 Gew.-% und besser 0,05 bis 1 Gew.-%, vorliegt.

15. Zusammensetzung nach einem der vorergehenden Ansprüche, **dadurch gekennzeichnet, dass** die lipophile Verbindung aus ätherischen Ölen und Ölen pflanzlicher Herkunft ausgewählt ist und in einem Gehalt im Bereich von 0,001 bis 5 Ges.-%, bezogen auf das Gesamtgewicht der Zusammensetzung, vorzugsweise 0,01 bis 2 Gew.-% und besser 0,05 bis 1 Ges.-%, vorliegt.

16. Verfahren zum Reinigen oder Abschminken von Keratinmaterialien, **dadurch gekennzeichnet, dass** man auf die Keratinmaterialien eine kosmetische Zusammensetzung nach einem der Ansprüche 1 bis 15 aufbringt.

## Claims

1. Cosmetic composition comprising:
- an aqueous phase comprising water in a content ranging from 30% to 95% by weight relative to the total weight of the composition,
- at least one lipophilic compound in a content ranging from 0.001% to 40% by weight relative to the total weight of the composition and chosen from antibacterial agents, antifungal agents, anti-seborrhoeic agents, antiacne agents, keratolytic agents, cicatrizing agents, pigmentation modifiers, tanning accelerators, artificial tanning agents, liporegulators, anti-ageing and anti-wrinkle agents, emollients, refreshing agents, vascular protectors, insect repellents, deodorants, antidandruff agents, hair-loss counteractants, essential oils, fragrances, sunscreens, antioxidants, free-radical scavengers and moisturizers, or fatty substances that are liquid at room temperature, fatty substances that are solid at room temperature or fatty substances that are semi-solid at room temperature, and mixtures thereof, and
- a surfactant system comprising the combination
- of at least one fatty acid ester of sucrose chosen from esters derived from the reaction of sucrose(s) and of fatty acid(s) comprising from 10 to 24 carbon atoms present in an amount ranging from 1% to 5% by weight relative to the total weight of the composition and
- of at least one fatty acid ester of polyglycerol chosen from esters derived from the reaction of polyglycerol comprising from 2 to 12 glycerol units, and of at least one fatty acid comprising less than 16 carbon atoms, present in an amount ranging from 1% to 5% by weight relative to the total weight of the composition,
the ratio of fatty acid ester of sucrose to fatty acid ester of polyglycerol ranging from 0.5 to 2, said composition comprising less than 1% additional surfactant.

2. Composition according to Claim 1, **characterized in that** the fatty acid ester of sucrose is chosen from esters derived from the reaction of sucrose(s) and of fatty acid(s) comprising from 12 to 20 carbon atoms, better still from 12 to 18 carbon atoms and even better still from 12 to 16 carbon atoms.

3. Composition according to Claim 1 or 2, **characterized in that** the fatty acid of the fatty acid ester of sucrose is chosen from oleic acid, lauric acid, palmitic acid, myristic acid, stearic acid, linoleic acid and capric acid, or mixtures thereof.

4. Composition according to any one of the preceding claims, **characterized in that** the fatty acid ester of sucrose is chosen from esters derived from the reaction of sucrose and of a fatty acid comprising from 12 to 18 carbon atoms and preferably from 12 to 16 carbon atoms.

5. Composition according to any one of the preceding claims, **characterized in that** the fatty acid ester of sucrose is chosen from sucrose laurate and sucrose palmitate, or a mixture thereof.

6. Composition according to any one of the preceding claims, **characterized in that** the fatty acid ester of polyglycerol is chosen from esters derived from the reaction of polyglycerol comprising from 3 to 10 glycerol units, and of at least one fatty acid comprising from 10 to 14 carbon atoms.

7. Composition according to Claim 6, **characterized in that** the fatty acid of the fatty acid ester of polyglycerol is chosen from lauric acid, myristic acid, capric acid and caprylic acid, or mixtures thereof.

8. Composition according to any one of the preceding claims, **characterized in that** the fatty acid ester of polyglycerol is chosen from esters derived from the reaction of polyglycerol comprising from 4 to 10 glycerol units, and of at least one fatty acid comprising less than 15 carbon atoms.

9. Composition according to any one of the preceding claims, **characterized in that** the fatty acid ester of polyglycerol is chosen from esters derived from the reaction of polyglycerol comprising from 4 to 10 glycerol units and of at least one fatty acid comprising from 8 to 12 carbon atoms and preferably from 10 to 12 carbon atoms, such as lauric acid and/or capric acid.

10. Composition according to any one of the preceding claims, **characterized in that** the fatty acid ester of polyglycerol is chosen from the ester derived from the reaction of polyglycerol-10 and Zauric acid, and the ester derived from the reaction of polyglycerol-4 and capric acid, and mixtures thereof.

11. Composition according to any one of the preceding claims, **characterized in that** the ratio of fatty acid ester of sucrose to fatty acid ester of polyglycérol ranges from 0.9 to 2.

12. Composition according to any one of the preceding claims, **characterized in that** the lipophilic compound is chosen from fragrances, essential oils, oils of plant origin, pasty fatty substances and butters, or mixtures thereof.

13. Composition according to any one of the preceding claims, **characterized in that** the lipophilic compound is present in a content ranging from 0.01% to 30% by weight and better still from 0.05% to 20% by weight relative to the total weight of the composition.

14. Composition according to any one of the preceding claims, **characterized in that** the lipophilic compound is chosen from essential oils, oils of plant origin, pasty fatty substances and butters and is present in a content ranging from 0.001% to 5% by weight, relative to the total weight of the composition, preferably from 0.01% to 2% by weight and better still from 0.05% to 1% by weight.

15. Composition according to any one of the preceding claims, **characterized in that** the lipophilic compound is chosen from essential oils and oils of plant origin and is present in a content ranging from 0.001% to 5% by weight, relative to the total weight of the composition, preferably from 0.01% to 2% by weight and better still from 0.05% to 1% by weight.

16. Process for cleansing or for removing makeup from keratin materials, **characterized in that** a cosmetic composition according to one of Claims 1 to 15 is applied to the said keratin materials.
